(19) Europäisches Patentamt
European Patent Office
Office européen des brevets

(11) **EP 3 238 680 A1**

(12) **EUROPÄISCHE PATENTANMELDUNG**

(43) Veröffentlichungstag:
01.11.2017 Patentblatt 2017/44

(21) Anmeldenummer: 17000291.9

(22) Anmeldetag: 23.02.2017

(51) Int Cl.:
*A61F 13/537* (2006.01)    *A61F 13/539* (2006.01)
*A61F 13/15* (2006.01)    *B32B 5/02* (2006.01)
*B32B 5/06* (2006.01)    *B32B 5/08* (2006.01)
*B32B 5/26* (2006.01)    *D04H 1/425* (2012.01)
*D04H 1/49* (2012.01)    *D04H 1/498* (2012.01)
*D04H 13/00* (2006.01)

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**MA MD**

(30) Priorität: **28.04.2016 DE 102016005158**

(71) Anmelder: **Sandler AG**
**95126 Schwarzenbach/Saale (DE)**

(72) Erfinder:
• **Die Erfinder haben auf ihr Recht verzichtet, als
solche bekannt gemacht zu werden.**

(54) **FLÜSSIGKEITSAUFNAHME UND -VERTEILVLIES FÜR HYGIENEARTIKEL**

(57)    Die vorliegende Erfindung handelt von einer Flüssigkeitsaufnahme und -verteillage für Hygieneartikel, welche eine Oberseite und eine Unterseite aufweist, wobei die Oberseite und die Unterseite aus Vliesstoffen bestehen, die durch Trockenverfahren gebildet wurden, die Fasermischung des die Unterseite bildenden Vliesstoffs weist einen geringeren mittleren Fasertiter auf als die Fasermischung des die Oberseite bildenden Vliesstoffs, der die Unterseite bildende Vliesstoff ist vorverfestigt und die Oberseite ist mit der Unterseite mechanisch verbunden.

EP 3 238 680 A1

**Beschreibung**

Wegwerfartikel für Hygieneanwendungen wie z.B. Damenbinden, Windeln sowie Inkontinenzprodukte haben den Zweck Körperflüssigkeiten so schnell wie möglich von der Haut wegzuleiten, um Hautirritationen zu vermeiden und dem Anwender ein sicheres und komfortables Gefühl zu geben. Für diesen Zweck sind Hygieneprodukte im Allgemeinen wie folgt aufgebaut:

- Erste Lage: Topsheet (Lochfolie oder Vlies)
- Zweite Lage: Acquisition and Distribution Layer, "ADL" (zumeist aus Vlies)
- Dritte Lage: Saugkern (bestehend aus Zellstoff und/oder Polyacrylsäure fixiert in einer oder mehreren Fixierlagen (SMS, ..)
- Abschlusslage: Folie, um ein Austreten der Flüssigkeit auf der Rückseite zu unterbinden

Die Lagen Topsheet, ADL und Saugkern müssen aufeinander abgestimmt sein, um eine Funktion im Hygieneprodukt zu gewährleisten.
Die wichtigste Rolle kommt dabei dem ADL als Transferlage zu. Dieser muss die Flüssigkeit vom Topsheet aufnehmen (Entwässerung des Topsheets), die Flüssigkeit in Längsrichtung des Produktes verteilen und kurz Zwischenspeichern (durch das Zwischenspeichern soll das sog. Gelblocking des Polyacrylsäure-Pulvers verhindert werden). Nach der kurzen Zwischenspeicherung wird die Flüssigkeit in den Saugkern zur finalen Speicherung weitergeleitet.
Der aktuelle Stand der Technik beschreibt als ADL-Materialien verschiedene Arten von Vliesstoffen.
Die wichtigsten Eigenschaften eines Aufnahme- und Verteilvlieses sind Dicke, Dichte und die daraus resultierenden Porengrößen sowie die Kapillarität. Durch diese Eigenschaften wird das Aufnahme- und Verteilverhalten maßgeblich beeinflusst.
Hier kommen je nach Produkt Airlaid-, Highloft- und Spunlacevliese zur Anwendung. In der Anmeldung DE 10 2012 015 219 A1 sind verschiedene Ausführungen von Spunlace-Vliesen beschrieben, die entsprechende Eigenschaften aufweisen.
Die im Stand der Technik beschriebenen Vliesarten sind vornehmlich Einlagenaufbauten mit homogen verteilten Fasertypen. Abhängig von der Herstellungsart kann jeweils nur eine benötigte Eigenschaft erzielt werden. So bieten z.B. Highloft-Vliese, d.h. rein thermisch mittels Schmelzfasern verfestigte Vliesstoffe, durch ihre Struktur eine hohe Dicke und eine damit verbundene gute Flüssigkeitsaufnahme. Durch die vergleichsweise hohe Porosität ist aber die Verteilwirkung sehr gering. Dadurch ist nur eine stark begrenzte Nutzung des darunter liegenden Saugkerns möglich.
Die durch das Airlaid-Verfahren hergestellten Vliese haben durch die feinen Kapillaren eine gute Verteilwirkung. Durch den hohen Zellstoff-Anteil ist aber die Dicke niedrig und die Flüssigkeit wird wieder an die Oberfläche abgegeben, was einen hohen Rewet zur Folge hat.
Zweilagenaufbauten, die aus einem einstufigen Prozess stammen, beispielsweise auf zwei Krempelanlagen hergestellte Vliesstoffe, wobei die Faserzusammensetzungen der einzelnen Krempelanlagen unterschiedlich sind, sind ebenfalls nicht zielführend. Da jede dieser Lagen die gleiche Verfestigungsenergie erfährt, kann entweder nur eine offene oder nur eine stark verdichtete Struktur dargestellt werden. Der graduelle Unterschied, der sich durch die unterschiedlichen Faserfeinheiten in den verschiedenen Lagen ergibt, ist nicht ausreichend um den Anforderungen in einem Hygieneprodukt gerecht zu werden.
Materialien nach dem Stand der Technik haben also keine klare Trennung der Eigenschaften Flüssigkeitsverteilung und Flüssigkeitsaufnahme. Sie kombinieren beide, wobei dadurch Abstriche in der jeweiligen Wirkung hingenommen werden müssen.
Die Aufgabe der vorliegenden Erfindung ist es daher, ein Material bereitzustellen, welches die genannten Nachteile des Standes der Technik vermeidet und eine verbesserte Flüssigkeitsaufnahme bei gleichzeitig gegebener Flüssigkeitsverteilung aufweist.
Gelöst wird die Aufgabe durch die im Anspruch 1 genannten Merkmale, sinnvolle Ausgestaltungen können den Ansprüchen 2 bis 11 entnommen werden.
Im Folgenden werden einige der in der Beschreibung verwendeten Begriffe näher definiert:

Textile Faserstoffe weisen zur Verbesserung der Verarbeitungseigenschaften aber auch zur Gewährleistung bestimmter Effekte im Fertigprodukt sogenannte "Verarbeitungshilfsmittel" auf der Faseroberfläche auf. Diese haften nur oberflächlich und werden beispielsweise bei einer Wasserstrahlverfestigung abgewaschen. Nach dem Abwaschen kommen ursprüngliche, durch das Faserpolymer bestimmte Fasereigenschaften wie statische Aufladung, Hydrophobie oder Hydrophilie wieder zum Tragen.

Eine "Saugfaser" im Sinne der vorliegenden Erfindung ist eine regenerierte Zellulosefaser, welche aus Lösungen von Zellulosederivaten hergestellt wird. Diese Viskosefaser kann eine Modifikation der Oberfläche haben, z.B. trilobaler Querschnitt, aber auch insgesamt so modifiziert sein, dass eine moderate Flüssigkeitsaufnahme bei gleichzeitig guter Flüssigkeitsspeicherung begünstigt wird. Erfindungsgemäße Vertreter sind beispielsweise Viscostar-Viskosefasern oder normale Viskosefasern des Herstellers Lenzing, aber auch die sogenannte Galaxy-Faser der Fa. Kehlheim Fibers. Im Besonderen können auch Viskosefasern welche nach dem Lyocell-Verfahren hergestellte werden (Tencel Fa. Lenzing) eingesetzt werden. Übliche Faserfeinheiten liegen im Bereich von 1,0 bis 3,3 dtex, bevorzugt 1,3 bis 2,2 dtex. Kommen Stapelfasern zum Einsatz, liegen die eingesetzten Faserlängen im Bereich von 10 - 70 mm, bevorzugt zwischen 35-50 mm. Die Saugfasern weisen handelsübliche Kräuselungen auf. Der Begriff Saugfaser kann sich aber auch, sollte die Unterseite ein Airlaid darstellen, auf Kurzfasern oder Pulp beziehen.

Der Begriff "Verteilfaser" wird im Sinne der vorliegenden Erfindung für in der Oberseite enthaltene Stapelfaserstoffe aus thermoplastischen und/oder duroplastischen Polymeren verwendet. Zum Einsatz kommen dabei Stapelfasern, die ohne Verarbeitungshilfsmittel auf der Oberfläche eine Sinkzeit von größer 5 Minuten auf-wiesen. Bevorzugt sind daher Fasern aus thermoplastischen Polymeren wie Polyester oder Polypropylen. Geeignet sind aber auch duroplastische Polymere wie beispielsweise Polyacrylfasern. Die Faserfeinheiten liegen im Bereich von 3,3 bis 17 dtex, bevorzugt 4,4 bis 10,0 dtex; die eingesetzten Faserlängen sind im Bereich von 10 - 80 mm, bevorzugt 35-60 mm. Die Verteilfasern weisen handelsübliche 2D- oder auch 3D-Kräuselungen auf.

Der Begriff "Füllfaser" wird im Sinne der vorliegenden Erfindung für Faserstoffe aus thermoplastischen und/oder duroplastischen Polymeren, welche im erfindungsgemäßen Vliesstoff innerhalb der Unterseite den Flüssigkeitstransport begünstigen, verwendet. Bevorzugt kommen dafür Fasern aus thermoplastischen Polymeren wie Polyester oder Polypropylen zur Anwendung. Geeignet sind aber auch duroplastische Polymere wie beispielsweise Polyacrylfasern zum Einsatz. Neben Vollfasern, d.h. Fasern die über ihren Querschnitt vollständig aus Polymer bestehen, können auch Hohlfasern oder Fasern mit profilierten Querschnitten, beispielsweise handelförmig, trilobal, dreieckig oder ähnliches eingesetzt werden. Die Faserfeinheiten liegen im Bereich von 0,8 bis 3,3 dtex, bevorzugt 1,0 bis 1,7 dtex; die eingesetzten Faserlängen sind im Bereich von 10 - 80 mm, bevorzugt 35-60 mm. Die Füllfasern weisen handelsübliche Kräuselungen auf.

"Bindefasern 1 und 2" im Sinne der vorliegenden Erfindung sind Fasern, die in einem erfindungsgemäß aufgebauten Produkt den Faserverbund stabilisieren, sodass ein weitestgehend dimensionsstabiles Vlies entsteht. Bindefasern sind homo- oder bikomponente, thermoplastische Polymerfaser, die schmelzbare Anteile aufweisen. Bei Beaufschlagung mit Temperatur schmelzen diese Anteile und stabilisieren nach dem Erkalten den Faserverbund. Erfindungsgemäß geeignete Fasern sind homopolymere Stapelfasern aus Co-Polyester, Co-Polyamid oder Polypropylen, erfindungsgemäß bevorzugt werden bikomponente Schmelzfasern in Kern-Mantel oder Side-by-Side Anordnung aus Kombinationen von niedrigschmelzendem Co-Polyester mit Polyester, Polyethylen mit Polypropylen oder Polyethylen mit Polyester. Die Faserfeinheiten für die in der Oberseite eingesetzten "Bindefasern 1" liegen im Bereich von 2,2 bis 12 dtex, bevorzugt 4,4 bis 6,7 dtex; die eingesetzten Faserlängen sind im Bereich von 10 - 80mm, bevorzugt 35-60mm. Die Faserfeinheiten für die in der Unterseite eingesetzten "Bindefasern 2" liegen im Bereich von 1,7 bis 3.3 dtex, bevorzugt 1,7 bis 2,2 dtex; die eingesetzten Faserlängen sind, bei Verwendung von Stapelfasern im Bereich von 10 - 80 mm, bevorzugt 35-60 mm, bei Verwendung von Kurzfasern, sollte die Unterseite durch ein Airlaid-Vlies gebildet werden, zwischen 1 bis 5 mm, bevorzugt 3 mm. Sollten die Bindefasern kardierbare Stapelfasern sein, so weisen sie handelsübliche Kräuselungen auf.

Hergestellt werden erfindungsgemäße Vliesstoffe mittels Trocken- oder Naßverfahren, die zugrundeliegenden Verfahren können dem im Jahr 2000 bei Wiley VCH-Verlag, Weinheim erschienenen Buch "Vliesstoffe" entnommen werden.

Speziell der die Unterseite bildende Vliesstoff kann ein luftgelegter Vliesstoff, ein sogenanntes "Airlaid" darstellen. Erfindungsgemäß bevorzugt werden kardierte Stapelfaservliesstoffe.

Die verwendeten Fasern werden bei der Erzeugung einer Fasermischung homogen miteinander vermischt. Die grundlegenden Techniken dazu sind ebenfalls im Buch "Vliesstoffe" aufgezeigt.

Als "Oberseite" wird erfindungsgemäß die Seite benannt, welche im Hygieneprodukt zum Benutzer hin gewandt ist und auf der Flüssigkeit zuerst auftrifft.

Die "Unterseite" ist die Seite des erfindungsgemäßen Vliesstoffes, die im Hygieneprodukt zum Saugkörper hin angebracht ist.

"Precursor" bezeichnet ein vorgefertigtes Vlies, welches bereits die Verfahrensschritte Fasermischung, Vliesbildung und Vliesverfestigung durchlaufen hat. Erfindungsgemäß wird die Unterseite durch einen Precursor gebildet.

Die nachfolgenden Beispiele beziehen sich, ohne darauf beschränkt zu sein, auf nach Trockenverfahren unter Verwendung des Kardierverfahrens hergestellte Stapelfaservliesstoffe.

Ergänzend dazu sind in Tabelle 1 die jeweils erzielten Prüfergebnisse aufgeführt. Die dabei ermittelten Parameter wurden gemäß den nachfolgend genannten Prüfmethoden ermittelt:

- Flächengewicht gemäß WSP 130.1., Angabe in kg/m$^2$
- Steighöhe gemäß WSP 010.1., Abschnitt 7.3, Angabe in mm

- Sinkzeit ungewaschen: zunächst wird eine homogene Fasermischung, beispielsweise mittels einer Laborkrempel hergestellt. Von der so hergestellten, kardierten Mischung werden 5 g entnommen und dem Sinkzeit-Test gemäß WSP 010.1, Abschnitt 7.1 unterzogen. Angabe in s
- Sinkzeit gewaschen: Herstellung der Fasermischung wie bei der "Sinkzeit ungewaschen". Danach wird zur Entfernung der Verarbeitungshilfsmittel von der Faseroberfläche die Fasermischung in demineralisiertem Wasser gewaschen, getrocknet und abschließend kardiert. Von der so hergestellten, kardierten Mischung werden 5 g entnommen und dem Sinkzeit-Test gemäß WSP 010.1, Abschnitt 7.1 unterzogen. Angabe in s.
- Dicke gemäß WSP 120.6, Abschnitt 7.2, Messdruck 0,5kPa, Angabe in m
- Dichte gemäß folgender Formel:

$$\text{Dichte (kg/m}^3) = \frac{\text{Flächengewicht (kg/m}^2)}{\text{Trockendicke (m)}}$$

- Strike Through gemäß WSP 70.3, Angabe in s
- Rewet gemäß WSP 80.10, Angabe in g
- Mittlerer Fasertiter innerhalb einer Lage: errechnet sich nach folgender Formel

$$\text{Mittlerer Fasertiter (dtex)} = \frac{A*\text{Titer 1} + B*\text{Titer 2} + C*\text{Titer 3}}{100}$$

A,B,C = Prozentanteil einer Faserkomponente in der Mischung, wobei die Summe aus A, B, und C = 100 ist.
Titer 1,2,3 = nominaler Titer der jeweiligen Faserkomponente in dtex

Die benötigten Eigenschaften, d.h. schnelle Flüssigkeitsaufnahme, gute Verteilung mit kurzzeitiger Zwischenspeicherung und Weiterleitung an den Saugkern werden erfindungsgemäß durch einen Aufbau aus zwei miteinander verbundenen Vlieslagen erzielt.

Ein erfindungsgemäß hergestelltes Flüssigkeitsaufnahme- und Verteilvlies durchläuft dabei folgende Verfahrensschritte:

- Herstellen einer Fasermischung für den Precursor, bestehend aus Saugfaser ggf. unter Zumischung von Füllfaser und / oder Bindefaser 2
- Herstellen einer unverfestigten Materialbahn aus der Fasermischung des Precursors
- Thermisches und/oder mechanisches Verfestigen der Materialbahn, sodass der Precursor mit einer Dichte von 80bis 120 kg/m$^3$ entsteht.
- Optional: Aufwickeln des Precursors
- Herstellen einer Fasermischung für die Oberseite, bestehend aus Verteilfaser, Bindefaser 1 und ggf. Saugfaser
- Herstellen einer unverfestigten Materialbahn aus der Fasermischung der Oberseite
- Ablegen der unverfestigten Materialbahn auf dem Precursor
- Verfestigen der Oberseite unter gleichzeitigem Verbinden der Oberseite mit dem Precursor mittels Wasserstrahlprozess
- Trocknen des Verbundes unter gleichzeitigem Auslösen der Bindefaser 1
- Wickeln des erfindungsgemäßen Flüssigkeitsaufnahme- und -verteilvlieses

Der die Unterseite bildende Vliesstoff besteht dabei erfindungsgemäß aus einem Precursor, welcher einen Gehalt von 60 bis 100 Massenprozent an Saugfaser aufweist. Je nach Ausführung des Precursors können bis zu 40 Massenprozent an Füllfasern eingebracht werden, welche die Flüssigkeitsweiterleitung an nachfolgende Speicherschichten begünstigen. Die Flüssigkeitsverteilwirkung kann verbessert werden, wenn die Füllfasern einen nicht-runden Faserquerschnitt aufweisen. Im Precursor werden Fasermischungen verwendet, deren mittlerer Fasertiter kleiner 3,3 dtex, bevorzugt kleiner 2,2 dtex und am meisten bevorzugt kleiner 2,0dtex liegt. Besteht der Precursor aus einem Airlaid-Material können zur Verfestigung des Airlaids bis zu 30 Massenprozent an Binderfaser 2 eingebracht werden.

Erfolgt die Herstellung des Precursors, wie erfindungsgemäß bevorzugt, als kardiertes Stapelfaservlies, ergibt sich eine Faserorientierung in Fertigungsrichtung des Vliesstoffes. Diese Orientierung ist für die spätere Verwendung vorteilhaft, da die Faserorientierung dann ebenfalls in Längsrichtung des fertigen Hygieneproduktes verläuft. Dadurch wird die Ausnutzung der zur Verfügung stehenden Saugfläche im Hygieneprodukt verbessert.

Bei der Verfestigung des Precursors, welche erfindungsgemäß bevorzugt, aber ohne darauf beschränkt zu sein, mittels Wasserstrahlen geschieht, erfolgt gleichzeitig eine Verdichtung des Precursors, sodass die Dichte des Precursors höher liegt als die des letztendlichen Aufnahme- und Verteilvliesstoffes.

Aufgrund des gewählten mittleren Fasertiters des Precursors in Verbindung mit der Verdichtung während der Verfestigung des Precursors wird die Kapillarwirkung und damit die Fähigkeit innerhalb des Precursors Flüssigkeit zu transportieren, positiv beeinflusst. Der Flüssigkeitstransport wird dabei mittels des Parameters Steighöhe beschrieben. Je höher diese nach 60 Sekunden ist, umso besser ist die Verteilwirkung eines Precursors.

Zum Erreichen von Steighöhen größer 50mm ist es notwendig, dass die Fasermischung für den Precursor bei einen mittleren Fasertiter von kleiner 3,3 dtex aufweist und der Precursor eine Dichte zwischen 80 kg/m$^3$ und 120 kg/m$^3$ hat. Liegt die Dichte unter 80 kg/m$^3$, dringt Flüssigkeit in die Unterseite ein, der Flüssigkeitstransport ist aber aufgrund schlechter Kapillarität nicht mehr gegeben. Liegt die Dichte über 120 kg/m$^3$ ist die Flüssigkeitsweiterleitung gestört, sodass auftreffende Flüssigkeit nicht mehr optimal an nachfolgende Speicherschichten weitergeleitet kann. Im Precursor wirkt sich weiterhin der Einsatz von Fasern mit nicht rundem Querschnitt positiv aus. Unter Berücksichtigung der Beibehaltung des mittleren Fasertiters von kleiner 3,3dtex können dabei auch Fasern mit Fasertitern bis zu 6.7dtex zum Einsatz kommen.

Erfindungsgemäß wird nun auf diesen Precursor ein unverfestigter, kardierter Faserflor abgelegt. Dieser Faserflor bildet nach einem daran anschließenden Verfestigungs- und Verbindungsschritt mittels Wasserstrahlen die Oberseite des erfindungsgemäßen Vliesstoffs.

Der Faserflor der Oberseite besteht dabei zu 35 bis 90 Massenprozent aus Verteilfasern und zu 10 bis 30 Massenprozent aus Bindefasern 1. In Abhängigkeit der zu handelnden Flüssigkeitsmenge kann die Notwendigkeit einer Saugfaser in der Oberseite bestehen. Findet das Fertigprodukt im Bereich Erwachseneninkontinenz Anwendung, so kann es notwendig sein, dass große Flüssigkeitsmengen, d.h. Mengen größer 250 ml pro Beaufschlagung zwischengespeichert werden müssen. In diesem Fall können auch in der Oberseite Saugfasern bis zu einem Anteil von maximal 35 Massenprozent vorgesehen werden.

Der mittlere Titer der Fasermischung der Oberseite liegt größer 3,3 dtex, bevorzugt im Bereich von 4,4 bis 12 dtex und besonders bevorzugt im Bereich von 6,7 bis 12dtex.
Durch diesen mittleren Fasertiter ist das, die Oberseite bildende Vlies, offen genug um Flüssigkeit rasch aufzunehmen und bis zur Weiterleitung an die Unterseite zwischen zu speichern. Je höher der mittlere Faserdurchmesser der Mischung ist, umso besser ist auch die Beständigkeit der Oberseite gegenüber Druckbelastungen. Der Rewet wird mit höheren mittleren Fasertitern positiv beeinflusst.

Durch eine thermische Aktivierung der Bindefasern 1 und - sofern verwendet - Bindefasern 2 wird dem Verbund zusätzliche Stabilität verliehen, die sowohl Vorteile im Verarbeitungsprozess an Hygieneproduktanlagen als auch zusätzliche Widerstandsfähigkeit gegenüber die bereits vorerwähnte Kompression in der Produktanwendung bietet.

Der Verbund zwischen dem Faserflor und dem Precursor geschieht erfindungsgemäß mittels Wasserstrahlvernadelung.

Diese Art der Verfestigung bewirkt neben dem Verbund der beiden Lagen auch, dass die Verarbeitungshilfsmittel auf den Oberflächen der eingesetzten Faserstoffe abgewaschen werden. Dadurch kommen die ursprünglichen Eigenschaften der den Fasern zugrundeliegenden Polymeren bei Kontakt mit wässrigen Flüssigkeiten wieder zum Tragen.

Nach dem Abwaschen der Verarbeitungshilfsmittel haben die Fasern bzw. die auf der Ober- bzw. Unterseite eingesetzte Fasermischung mehr oder weniger hydrophile Eigenschaften, welche über die Sinkzeit beschrieben werden.

Fasern aus verschiedenen Grundpolymeren haben, solange die Verarbeitungshilfsmittel noch auf der Faseroberfläche liegen, vergleichbare Sinkzeiten. Für die untersuchten Faserstoffe Polyester, Polypropylen und Viskose lagen die Sinkzeiten ohne Vorwaschen im Bereich von 2 bis 10 Sekunden.
Wäscht man die Verarbeitungshilfsmittel vor dem Test ab, zeigt sich ein deutlicher Unterschied. Viskosefasern liegen weiterhin im Bereich von 2 Sekunden, wohingegen bei Polypropylen-Fasern die Sinkzeit auf über 1000 Sekunden und bei PolyesterFasern auf rund 600 Sekunden ansteigt.

Überraschenderweise wurde herausgefunden, dass bei der Mischung von Verteilfasern mit Saugfasern die Sinkzeit nicht auf den Wert der Saugfaser fällt, sondern in sich ein Zwischenwert einstellt, welcher abhängig vom Gehalt an Saugfaser ist. Je geringer der Gehalt an Saugfaser ist, umso höher ist die Sinkzeit einer daraus hergestellten und gewaschenen Fasermischung. Je hydrophober eine Mischung sein soll, umso geringer muss der Gehalt an Saugfasern sein.

Es ist erfindungsgemäß beabsichtigt, auf der Oberseite Fasermischungen zu verwenden, welche im gewaschenen Zustand Sinkzeiten größer 250 Sekunden aufweisen.

Für die Unterseite, das heißt für den Precursor, kommen Fasermischungen zum Einsatz, welche im gewaschenen Zustand Sinkzeiten von kleiner 200 Sekunden aufweisen.

Der Precursor wird zusammen mit dem groben Faserflor einer Wasserstrahlverfestigung zugeführt. Durch die Wasserstrahlen wird einerseits der Faserflor der Oberseite verfestigt, andererseits werden beide Lagen - Precursor und Oberseite miteinander verbunden.

Dabei kann die Verfestigung vollflächig stattfinden, es können aber auch nur Teilbereiche mit Wasserstrahlen beaufschlagt werden oder es wird mit Strukturtrommeln gearbeitet zur Einbringung einer das Flüssigkeitshandling begünstigenden Struktur auf der Oberseite.

So lässt sich beispielsweise eine Linienstruktur in Fertigungsrichtung erzeugen, bei welcher sich quer zur Fertigungsrichtung Bereiche, in welchen Precursor und Oberseite mit einander verbunden sind, mit Bereichen abwechseln, in welchen Precursor und Oberseite nicht verbunden sind. Auch das Einbringen einer Lochstruktur, die regelmäßig über die Oberfläche verteilt sind, ist erfindungsgemäß vorsehbar.

Diese Art des Aufbaus hat Vorteile, wenn sich bei späterem Einsatz, beispielsweise in Artikel für Erwachsenen-Inkontinenz, hohe Kompressionen ergeben. Hier können höhere Fasertiter in der Fasermischung der Oberseite positiven Einfluss auf das Rewet- und Strike-Through-Verhalten unter Belastung nehmen.

Ein so hergestelltes, erfindungsgemäß ausgeführtes Flüssigkeitsaufnahme- und Verteilvlies hat aufgrund des im Vergleich zur Unterseite höheren mittleren Fasertiters in der Fasermischung der Oberseite, eine offenere Struktur auf der Oberseite. Dies ermöglicht eine rasche Flüssigkeitsaufnahme, was sich in geringen Strike Through-Zeiten zeigt.

Bedingt durch die geringere Benetzungsfähigkeit der Fasermischung der Oberseite nach dem Abwaschen der Verarbeitungshilfsmittel wird die Flüssigkeit aber nur zwischengelagert, es erfolgt kein oder nur ein geringes Speichern in der Oberseite. Der Rewet ist entsprechend gering.

Aufgrund der Verwendung eines im Vergleich zur Oberseite geringeren Fasertiters in der Fasermischung der Unterseite einhergehend mit einer höheren Dichte der Unterseite, wird auftreffende Flüssigkeit schnell innerhalb der Unterseite verteilt.

Die Fasermischung der Unterseite weist im gewaschenen Zustand erfindungsgemäß eine im Vergleich zur Fasermischung der Oberseite geringere Sinkzeit auf.

Betrachtet man die Tabelle 1, so haben alle Fasermischungen im ungewaschenen Zustand eine Sinkzeit im Bereich von 2 bis 6 Sekunden. Nach dem Waschen zeigen sich jedoch deutliche Unterschiede.

Die Sinkzeit der Fasermischung der Unterseite bleibt nach dem Waschen bei Verwendung eines hohen Anteils an Saugfasern im Bereich von 2 bis 6 Sekunden, je mehr Füllfasern eingebracht werden, umso höher wird die Sinkzeit. Dabei werden Sinkzeiten von kleiner 200 Sekunden angestrebt, um die Flüssigkeitsspeicherung, aber die Flüssigkeitsverteilung weiterhin zu gewährleisten. Ein Gehalt von 40 Massenprozent Füllfasern wird daher als maximal angesehen.

Die Sinkzeit der Fasermischung der Oberseite steigt nach dem Waschen deutlich an, sodass diese größer 300 Sekunden liegt. Dies ist notwendig, damit Flüssigkeit nur temporär in der Oberseite eingelagert aber nicht gespeichert wird

Die Beimischung von Füllfasern in der Fasermischung des Precursors begünstigt dessen Durchlässigkeit gegenüber Flüssigkeiten, speziell bei Auftreffen hoher Flüssigkeitsmengen. Fertigt man den Precursor rein aus Saugfasern hat dieser eine hohe Flüssigkeitsverteilwirkung, ausgedrückt als Steighöhe. Bedingt durch das Aufquellen der Saugfasern bei Flüssigkeitsaufnahme verringert sich allerdings die Durchlässigkeit für Flüssigkeiten und die Weiterleitung an nachfolgende Speicherschichten wird gestört. Das Einbringen von nicht quellenden Füllfasern vermindert diesen Effekt, sodass auch bei großen Flüssigkeitsmengen (größer 250 ml) eine gute Flüssigkeitsweiterleitung erzielt wird. Dies geht allerdings zu Lasten der Verteilwirkung. Ein Anteil von Füllfasern und / oder Bindefasern 2 in Höhe von 40% in der Fasermischung des Precursor stellt daher die Obergrenze dar.

Aufgrund der Tatsache, dass der Precursor bereits eine Verfestigung erfahren hat, kommt es beim Verbinden des Precursors mit dem Faserflor der Oberseite nicht zu einer ungewünschten, prozessbedingten Vermischung der Fasern aus beiden Lagen was

- in der Oberlage nicht zu einer ungewollten Anreicherung von Saugfasern und
- in der Unterlage nicht zu einer ungewollten Anreichung von Verteil- oder Bindefasern führt.

Nachweislich wird eine deutlich gesteigerte Verteilwirkung (Wicking gegen die Schwerkraft) und somit eine verbesserte Weitergabefunktion über die gesamte Fläche des Saugkerns erreicht.

Durch eine thermische Aktivierung der Schmelzfaser wird dem Verbund zusätzliche Stabilität verliehen, die sowohl Vorteile im Verarbeitungsprozess an Hygieneproduktanlagen als auch zusätzliche Widerstandsfähigkeit gegenüber Kompression in der Produktanwendung bietet.

Betrachtet man Tabelle 1 so kann man folgendes erkennen:

Die in der Tabelle 1 genannten Vliesstoffe bestehen aus Stapelfasern, welche kardiert und anschließend mittels Wasserstrahlen verfestigt wurden. Dabei wurden die Fasern der einzelnen Fasermischungen zunächst mit konventionellen Aggregaten homogen gemischt. Die so hergestellten Fasermischungen werden dann einem konventionellen Kardierverfahren zugeführt. Entsprechende Technik kann dem Buch "Vliesstoffe", Wiley VCH, 2000 entnommen werden.

Nach erfolgter Wasserstrahlverfestigung und Wasserstrahlverbund bei den erfindungsgemäß ausgeführten Materialien erfolgt abschließend eine Trocknungsbehandlung. Entsprechende Technik kann dem Buch "Vliesstoffe", Wiley VCH, 2000 entnommen werden.

Bei dieser Behandlung wird der Temperaturverlauf im Trockenaggregat so gewählt, dass zunächst die Feuchtigkeit im Vlies verdampft wird und abschließend die in der Mischung enthaltene Bindefasern je nach Polymerzusammensetzung bei Temperaturen größer 120°C aktiviert wird. Das Vlies wird dadurch zusätzlich verfestigt.

Ein dem Stand der Technik entsprechendes einlagiges Material, welches einen homogenen Aufbau aufweist zeigt Steighöhen von 19 mm, Rewet-Werte von 1,4 g und einen Strike Through von 2,2 s. Dies bei einem mittleren Fasertiter der Fasermischung von 4,4 dtex. Durch die Wahl des mittleren Titers wird die Flüssigkeit gut verteilt und schnell aufgenommen, allerdings aufgrund der geringen Steighöhe nur schlecht verteilt.

Ein nach dem Stand der Technik hergestelltes Produkt aus zwei unterschiedlichen Lagen, die gleichzeitig verfestigt und miteinander verbunden werden, zeigt zwar eine gute Ansaugzeit in Verbund mit guten Rewet, allerdings ist die Steighöhe mit 22 mm nur ungenügend, in der Folge ebenso die Verteilwirkung ungenügend.

Ein erfindungsgemäß ausgeführtes Material 1 zeichnet sich durch deutlich verbesserte Steighöhe 75 mm bei gleichzeitig geringem Rewet und Strike Through aus. Bei dem Ausführungsbeispiel 1 wurde die Fasermischung des Precursors aus 100 Massenprozent Saugfaser gewählt. Die Sinkzeit dieser Mischung liegt im gewaschenen Zustand bei1,9 s, der mittlere Fasertiter bei 1,7 dtex.

In einem vorgeschalteten Fertigungsschritt wurde die Fasermischung kardiert, sodass ein Faserflor von 0,030 kg/m$^2$ Flächengewicht entstand. Dieser wurde während der nachgeschalteten Verfestigung mittels Wasserstrahlen von 120 bar Druck auf eine Dicke von 0,00035 m, entsprechend einer Dichte von 85,7 kg/m$^3$ verdichtet, sodass der Precursor entstand.

Dieser hergestellte Precursor wurde erneut der Produktionsanlage vorgelegt. Dabei wurde auf die Oberseite des Precursors eine Lage kardiertes Vlies in einem Gewicht von 0,038kg/m$^2$ abgelegt. Die Fasermischung wurde dabei so gewählt, dass sich eine Sinkzeit im gewaschenen Zustand ergibt, die mit 347 Sekunden deutlich über der der Mischung des Precursors liegt. Der mittlere Fasertiter auf der Oberseite liegt bei 4,9dtex.

Der so entstandene Lagenaufbau wurde dann erneut einer Wasserstrahlverfestigung mit einem Druck von 110bar zugeführt. Dabei wurde das bisher unverfestigte Vlies der Oberseite verfestigt und gleichzeitig auch mit der Unterseite verbunden. In der Folge entstand das erfindungsgemäße Ausführungsbeispiel 1.

Wie man der Tabelle 1 entnehmen kann, ist die Dichte des Gesamtaufbaus mit 69kg/m$^3$ vergleichbar mit aus dem Stand der Technik bekannten Materialien. Durch die Verwendung eines verdichteten, vorgefertigten und saugfähigen Precursors auf der Unterseite wurde die Flüssigkeitsverteilwirkung, ausgedrückt als Steighöhe um den Faktor 3 verbessert. Durch die Kombination mit der eine höhere Sinkzeit und mittleren Fasertiter aufweisenden Fasermischung der Oberseite wurde dabei das Rewet- und Strike-Through-Verhalten des Stands der Technik beibehalten.

Das Ausführungsbeispiel 2 zeigt den Einfluss der Fasermischung der Unterseite. Bei gleicher Mischung für die Oberseite wurden in die Unterseite Füllfasern mit einem Anteil von 40 Massenprozent eingemischt. Der mittlere Fasertiter von 1,7 dtex wurde beibehalten. Die Sinkzeit dieser Mischung liegt im gewaschenen Zustand bei 182s, der mittlere Fasertiter bei 1,7 dtex.

In einem vorgeschalteten Fertigungsschritt wurde die Fasermischung der Unterseite kardiert, sodass ein Faserflor mit einem Flächengewicht von 0,030 kg/m$^2$ entstand. Dieser wurde während der nachgeschalteten Verfestigung mittels Wasserstrahlen von 130 bar Druck auf eine Dicke von 0,00032m, entsprechend einer Dichte von 93,8 kg/m$^3$ verdichtet.

Dieser so hergestellte Precursor wurde erneut der Produktionsanlage vorgelegt. Dabei wurde auf die Oberseite des Precursors eine Lage kardiertes Vlies in einem Gewicht von 0,040kg/m$^2$ abgelegt. Die Sinkzeit der Fasermischung im gewaschenen Zustand liegt bei 320 Sekunden, wohingegen die Mischung des Precursors bei 182 Sekunden liegt. Der mittlere Fasertiter auf der Oberseite liegt bei 4,9dtex.

Der so entstandene Lagenaufbau wurde dann erneut einer Wasserstrahlverfestigung mit einem Druck von 110bar zugeführt. Dabei wurde das bisher unverfestigte Vlies der Oberseite verfestigt und gleichzeitig auch mit der Unterseite verbunden. In der Folge entstand das erfindungsgemäße Vlies des Ausführungsbeispiels 2.

Wie man der Tabelle 1 entnehmen kann, ist die Dichte des Gesamtaufbaus mit 70kg/m$^3$ vergleichbar mit aus dem Stand der Technik bekannten Materialien. Durch die Verwendung eines verdichteten Precursors mit Füllfasern auf der Unterseite wurden im erfindungsgemäßen Vlies 2 sowohl der StrikeThrough als auch der Rewet gegenüber dem erfindungsgemäßen Vlies 1 verbessert. Dies ohne die Steighöhen zu beeinträchtigen.

Das Ausführungsbeispiel 3 zeigt den Einfluss der Fasermischung der Oberseite. Bei mit erfindungsgemäßem Bespiel 2 gleicher Mischung für die Unterseite wurde in der Oberseite auf den Anteil an Saugfasern verzichtet. Gleichzeitig wurde der mittlere Fasertiter auf 6,5dtex angehoben. Die Sinkzeit dieser Mischung liegt aufgrund des Fehlens jeglicher saugfähigen Komponente im gewaschenen Zustand bei 784 Sekunden.

In einem vorgeschalteten Fertigungsschritt wurde für das Ausführungsbeispiel 3 die Fasermischung der Unterseite kardiert, sodass ein Faserflor von 0,032 kg/m$^2$ Flächengewicht entstand. Dieser wurde während der nachgeschalteten Verfestigung mittels Wasserstrahlen von 130 bar Druck auf eine Dicke von 0,00029 m, entsprechend einer Dichte von 110 kg/m$^3$ verdichtet, sodass der Precursor entstand.

Dieser so hergestellte Precursor wurde erneut der Produktionsanlage vorgelegt. Dabei wurde auf die Oberseite des Precursors eine Lage kardiertes Vlies in einem Flächengewicht von 0,043 kg/m$^2$ abgelegt

Der so entstandene Lagenaufbau wurde dann erneut einer Wasserstrahlverfestigung mit einem Druck von 110 bar zugeführt. Dabei wurde das bisher unverfestigte Vlies der Oberseite verfestigt und gleichzeitig auch mit der Unterseite verbunden. In der Folge entstand das erfindungsgemäße Ausführungsbeispiel 3.

Wie man der Tabelle 1 entnehmen kann, ist die Dichte des Gesamtaufbaus mit 68 kg/m$^3$ vergleichbar mit aus dem Stand der Technik bekannten Materialien. Durch die Verwendung einer Fasermischung mit im Vergleich zur Fasermischung des Precursors deutlich höherem mittleren Fasertiter und auch höherer Sinkzeit, wurde das Rewet- und Strike-Through-Verhalten gegenüber den erfindungsgemäßen Beispiel 1 und 2 bei Beibehaltung der Steighöhen nochmals verbessert.

| Parameter | Einheit | Stand der Technik einlagig | Stand der Technik zweilagig in einem Lauf verbunden | Erfindungsgemäßes Vlies 1 | Erfindungsgemäßes Vlies 2 | Erfindungsgemäßes Vlies 3 |
|---|---|---|---|---|---|---|
| Fasermischung Oberseite | Massenprozent | * 40% Verteilfaser PET 6,7<br>* 25% Bindefaser PET/CoPET 4,4<br>* 35% CV 1,7 | * 70% Verteilfaser PET 6,7 dtex<br><br>* 30% Bindefaser PET/CoPET 4,8 dtex | * 33% Verteilfaser PET 7dtex<br><br>* 33% Bindefaser PET/CoPET 4,4dtex<br><br>* 33% Saugfaser CV 3,3 dtex | * 33% Verteilfaser PET 7dtex<br><br>* 33% Bindefaser 1 PET/CoPET 4,4dtex<br><br>* 33% Saugfaser CV 1,7 dtex | *90% Verteilfaser PET 6,7 dtex<br><br>* 10% Bindefaser 1 PET/CoPET 4,8 dtex |
| Fasermischung Unterseite | Massenprozent | | * 100% Saugfaser CV 1,7 dtex | * 100% Saugfaser CV 1,7 dtex | * 60% Saugfaser CV 1,7dtex<br>* 40% Füllfaser PET 1,7dtex | * 60% Saugfaser CV 1,7dtex<br>* 40% Füllfaser PET 1,7dtex |
| Flächengewicht gesamt | kg/m$^2$ | 0,053 | 0,060 | 0,068 | 0,070 | 0,075 |
| Flächengewicht Precursor | kg/m$^2$ | | | 0,030 | 0,030 | 0,032 |
| Dicke Precursor | m | | | 0,00035 | 0,00032 | 0,00029 |
| Dicke gesamt | m | 0,00082 | 0,00090 | 0,00099 | 0,00100 | 0,00110 |
| Dichte Precursor | kg/m$^3$ | | | 85,7 | 93,8 | 110,3 |
| Dichte gesamt | kg/m$^3$ | 64,6 | 66,7 | 68,7 | 70,0 | 68,2 |
| Mittlerer Fasertiter Unterseite | dtex | | 1,7 | 1,7 | 1,7 | 1,7 |
| Mittlerer Fasertiter Oberseite | dtex | 4,4 | 6,1 | 4,9 | 4,9 | 6,5 |
| Steighöhe Precursor | mm | | | 62 | 54 | 55 |
| Steighöhe gesamt | mm | 19 | 22 | 75 | 72 | 68 |
| Rewet | g | 1,4 | 0,8 | 1,1 | 1,3 | 1,3 |

(fortgesetzt)

| Parameter | Einheit | Stand der Technik einlagig | Stand der Technik zweilagig in einem Lauf verbunden | Erfindungsgemäßes Vlies 1 | Erfindungsgemäßes Vlies 2 | Erfindungsgemäßes Vlies 3 |
|---|---|---|---|---|---|---|
| StrikeThrough | s | 2,2 | 0,1 | 1,8 | 1,4 | 1,1 |
| Sinkzeit der Fasermischung Oberseite ungewaschen | s | | 2,9 | 4,8 | 5,2 | 3,2 |
| Sinkzeit der Fasermischung Oberseite gewaschen | s | | 561 | 347 | 320 | 784 |
| Sinkzeit der Fasermischung Unterseite ungewaschen | s | | 1,8 | 2,0 | 2,6 | 2,5 |
| Sinkzeit der Fasermischung Unterseite gewaschen | s | | 1,9 | 1,9 | 182 | 183 |

**Patentansprüche**

1. Flüssigkeitsaufnahme und -verteilvlies für Hygieneartikel, welches eine Oberseite und eine Unterseite aufweist **dadurch gekennzeichnet, dass**

   - die Oberseite und die Unterseite aus Vliesstoffen bestehen, die durch Trockenverfahren gebildet wurden,
   - die Fasermischung des die Unterseite bildenden Vliesstoffs einen geringeren mittleren Fasertiter aufweist als die Fasermischung des die Oberseite bildenden Vliesstoffes,
   - der die Unterseite bildende Vliesstoff vorverfestigt ist und,
   - die Oberseite mit der Unterseite mechanisch verbunden ist.

2. Flüssigkeitsaufnahme und -verteilvlies gemäß Anspruch 1, **dadurch gekennzeichnet, dass**

   - die Fasermischung des die Unterseite bildenden Vliesstoffs im gewaschenen Zustand eine geringere Sinkzeit aufweist als die Fasermischung des die Oberseite bildenden Vliesstoffs im gewaschenen Zustand, wobei die Sinkzeit gemäß der in der Beschreibung genannten Methode ermittelt wird

3. Flüssigkeitsaufnahme und -verteilvlies gemäß einem der vorhergehenden Ansprüche, **dadurch gekennzeichnet, dass**

   - der die Oberlage bildende Vliesstoff ein Stapelfaservliesstoff ist.

4. Flüssigkeitsaufnahme und -verteilvlies gemäß einem der vorhergehenden Ansprüche, **dadurch gekennzeichnet, dass**

   - der die Unterseite bildende vorverfestigte Vliesstoff eine Dichte im Bereich von 80 bis 120 kg/m$^3$ aufweist.

5. Flüssigkeitsaufnahme und -verteilvlies gemäß einem der vorhergehenden Ansprüche, **dadurch gekennzeichnet, dass**

   - der die Unterseite bildende Vliesstoff ein vorverfestigter, wasserstrahlverfestiger Vliesstoff ist.

6. Flüssigkeitsaufnahme und -verteilvlies gemäß einem der vorhergehenden Ansprüche, **dadurch gekennzeichnet, dass**

   - der Verbund zwischen Ober- und Unterseite mechanisch mittels Wasserstrahlen hergestellt wird.

7. Flüssigkeitsaufnahme und -verteilvlies gemäß einem der vorhergehenden Ansprüche, **dadurch gekennzeichnet, dass**

   - der die Oberseite bildende Vliesstoff aus 35 - 90 Massenprozent einer Verteilfaser, aus 10 - 30 Massenprozent einer Bindefaser 1 und 0 - 35 Massenprozent einer Saugfaser besteht.

8. Flüssigkeitsaufnahme und -verteilvlies gemäß einem der vorhergehenden Ansprüche, **dadurch gekennzeichnet, dass**

   - der die Unterseite bildende Vliesstoff aus 0-100 Massenprozent einer Saugfaser, zwischen 0 und 40 Massenprozent aus einer Füllfaser und zwischen 0-30 Massenprozent aus einer Bindefaser 2 besteht.

9. Flüssigkeitsaufnahme und -verteilvlies gemäß einem der vorhergehenden Ansprüche, **dadurch gekennzeichnet, dass**

   - der die Unterseite bildende Vliesstoff aus einem in einem separaten Herstellungsprozess gefertigten Vorprodukt besteht.

10. Flüssigkeitsaufnahme und -verteilvlies gemäß einem der vorhergehenden Ansprüche, **dadurch gekennzeichnet, dass**

- die Oberseite mit der Unterseite vollflächig mechanisch verbunden ist.

11. Flüssigkeitsaufnahme und -verteilvlies gemäß einem der vorhergehenden Ansprüche 1 bis 9, **dadurch gekennzeichnet, dass**

- die Oberseite mit der Unterseite nur teilweise mechanisch verbunden ist.

**Europäisches Patentamt**
**European Patent Office**
**Office européen des brevets**

# EUROPÄISCHER RECHERCHENBERICHT

Nummer der Anmeldung

EP 17 00 0291

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2014/121626 A1 (FINN NIALL [AU] ET AL) 1. Mai 2014 (2014-05-01) | 1,3,5-7, 9-11 | INV. A61F13/537 |
| Y | * Abbildung 5 * * Absätze [0102] - [0107]; Beispiele 6s.1-6S.4; Tabelle 2 * * Absatz [0083] * * Absätze [0001], [0123], [0126] - [0127] * * Absätze [0092], [0093], [0095]; Abbildung 4 * | 2,8 | A61F13/539 A61F13/15 B32B5/02 B32B5/06 B32B5/08 B32B5/26 D04H1/425 D04H1/49 D04H1/498 |
| X | ----- US 6 022 818 A (WELCHEL DEBRA NELL [US] ET AL) 8. Februar 2000 (2000-02-08) | 1,3,6,10 | D04H13/00 |
| Y | * Spalte 15, Zeile 60 - Spalte 16, Zeile 16 * * Spalte 15, Zeile 1 - Zeile 15 * | 2,8 | |
| A | ----- WO 2007/077214 A1 (JOHNSON & JOHNSON GMBH [DE]; GARCIA ALVARO [ES]; KIRSCH ELISABETH [DE]) 12. Juli 2007 (2007-07-12) * das ganze Dokument * ----- | 1-11 | |

RECHERCHIERTE SACHGEBIETE (IPC)

A61F
B32B
D04H

Der vorliegende Recherchenbericht wurde für alle Patentansprüche erstellt

| Recherchenort | Abschlußdatum der Recherche | Prüfer |
|---|---|---|
| München | 1. September 2017 | Beckert, Audrey |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

                      

& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 17 00 0291

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

01-09-2017

| Im Recherchenbericht angeführtes Patentdokument | Datum der Veröffentlichung | Mitglied(er) der Patentfamilie | Datum der Veröffentlichung |
|---|---|---|---|
| US 2014121626 A1 | 01-05-2014 | AU 2013340407 A1<br>CN 104769173 A<br>EP 2914766 A1<br>KR 20150081305 A<br>MX 341237 B<br>NZ 708476 A<br>US 2014121626 A1<br>WO 2014068492 A1 | 11-06-2015<br>08-07-2015<br>09-09-2015<br>13-07-2015<br>09-08-2016<br>28-04-2017<br>01-05-2014<br>08-05-2014 |
| US 6022818 A | 08-02-2000 | AU 703499 B2<br>BR 9608569 A<br>CA 2220725 A1<br>CN 1192250 A<br>DE 69624703 D1<br>DE 69624703 T2<br>EP 0830470 A2<br>KR 19990022489 A<br>MX 206776 B<br>US 6022818 A<br>WO 9641045 A2 | 25-03-1999<br>29-12-1998<br>19-12-1996<br>02-09-1998<br>12-12-2002<br>20-03-2003<br>25-03-1998<br>25-03-1999<br>18-02-2002<br>08-02-2000<br>19-12-1996 |
| WO 2007077214 A1 | 12-07-2007 | DE 102006000780 A1<br>WO 2007077214 A1 | 05-07-2007<br>12-07-2007 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

**In der Beschreibung aufgeführte Patentdokumente**

- DE 102012015219 A1

**In der Beschreibung aufgeführte Nicht-Patentliteratur**

- Vliesstoffe. Wiley VCH, 2000